# EUROPEAN PATENT SPECIFICATION

(11) **EP 0 566 288 B1**
(45) Date of publication and mention of the grant of the patent: **02.08.1995**
(21) Application number: 93302598.3
(22) Date of filing: 01.04.1993
(51) Int. Cl.: C07D 311/30, C07D 405/06, C07D 409/12, A61K 31/35, A61K 31/47, A61K 31/385

(54) **Flavone derivatives**
Flavonderivate
Dérivés de flavone

(30) Priority: 10.04.1992 IT MI920884
(43) Date of publication of application: 20.10.1993
(73) Proprietor: RECORDATI S.A. CHEMICAL and PHARMACEUTICAL COMPANY, 6830 Chiasso (CH); RECORDATI INDUSTRIA CHIMICA E FARMACEUTICA S.p.a., 20148 Milano (IT)
(72) Inventor: Leonardi, Amedeo, I-20154 Milano (IT); Motta, Gianni, I-20030 Barlassina (IT); Riva, Carlo, I-21100 Varese (IT); Guarneri, Luciano, I-20024 Garbagnate Milanese (IT)
(74) Representative: SERJEANTS

(56) References cited:
- EP-A- 0 072 620
- EP-A- 0 108 986
- US-A- 3 891 651
- ARZNEIMITTEL FORSCHUNG vol. 43, no. 1, 1993, pages 28 - 34 'New basic esters of 2-pheny l-3-methyl-4-oxo-4H-1-benzopyran-8-carboxy lic acid endowed with spasmolytic properties'
- CHEMICAL ABSTRACTS, vol. 106, 1987, Columbus, Ohio, US; abstract no. 27559h, 'Effects of 3-methyl 4-oxo-2-phenyl-N-Ä2-(1-piperidinyl)ethylÜ 4H-1-benzopyran- 8-carboxamide hydrochloride monohydrate.' page 34 ; & NIPPON YAKURIGAKU ZASSHI vol. 88, no. 5, 1986, pages 355-361
- PATENT ABSTRACTS OF JAPAN vol. 14, no. 086 (C-0690)19 February 1990 & JP-A-12 99 284

## Description

The invention relates to flavone derivatives, and to pharmaceutical compositions containing them.

Urinary incontinence is a pathological condition affecting an increasing number of elderly people. This disease is currently treated with anticholinergic-spasmolytic drugs. One such drug, which is distinguished by its practical absence of anticholinergic effects, is 8-(2-piperidinoethoxycarbonyl)-3-methyl-4-oxo-2-phenyl-4H-1-benzopyran, commonly known as Flavoxate. It has the formula 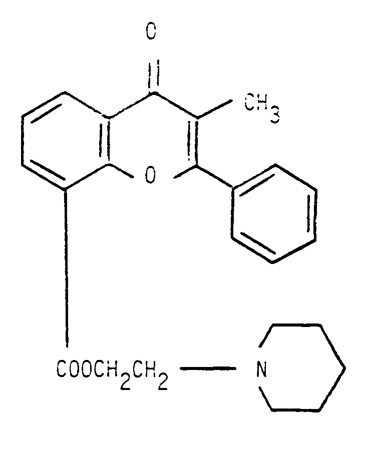
The use of this drug is however hampered by its low stability in physiological fluids.

The compounds of the invention, described below, essentially include methoxyphenyl substituted complex amino moieties in place of the piperidino group. A further change comprises alternatives to the ethoxycarbonyl group which spaces the amino moiety from the benzopyran ring. The compounds of the invention have been found to exhibit powerful antispasmodic action, and to be considerably more stable at physiological pH than Flavoxate, so that the half-life of the drug is prolonged. Their use as long lasting antispasmodic drugs is therefore indicated, with particular emphasis for the treatment of disturbances of the lower urinary tract, without limiting their use to this field.

### COMPOUNDS OF THE INVENTION

The compounds of the invention have the general formula I 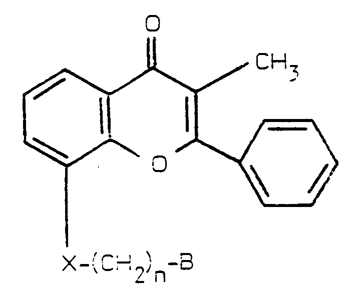
wherein
- X: represents one of the following groups, each of which is depicted with its left hand end being the end which attaches to the benzopyran ring and its right hand end being the end which attaches to the alkylene chain:
(X1) -CO-,
(X2) -COO-,
(X3) -CONH-,
(X4) -CON(CH₃)-,
(X5) -CH₂NH-,
(X6) -CH₂N(CH₃)-,
(X7) -CH₂NHCO-,
(X8) -CH₂N(CH₃)CO-,
(X9) -CH₂O-,
(X10) -CH₂S-,
(X11) -CH₂SO-, and
(X12) -CH₂SO₂-,
- n: is an integer of from 1 to 4; and
- B: represents one of the following groups: 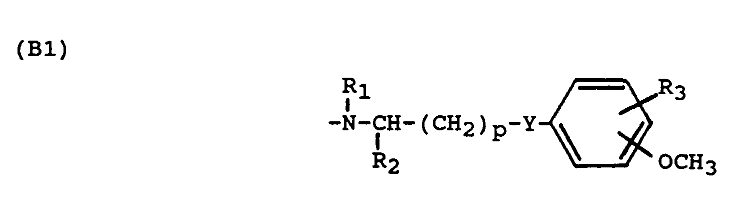 wherein R₁ represents an alkyl group having from 1 to 4 carbon atoms, R₂ represents a hydrogen atom or an alkyl group having from 1 to 4 carbon atoms, R₃ represents a hydrogen atom or a methoxy group, p is an integer of from 1 to 3, and Y represents (Y1) a valence bond or one of the following groups 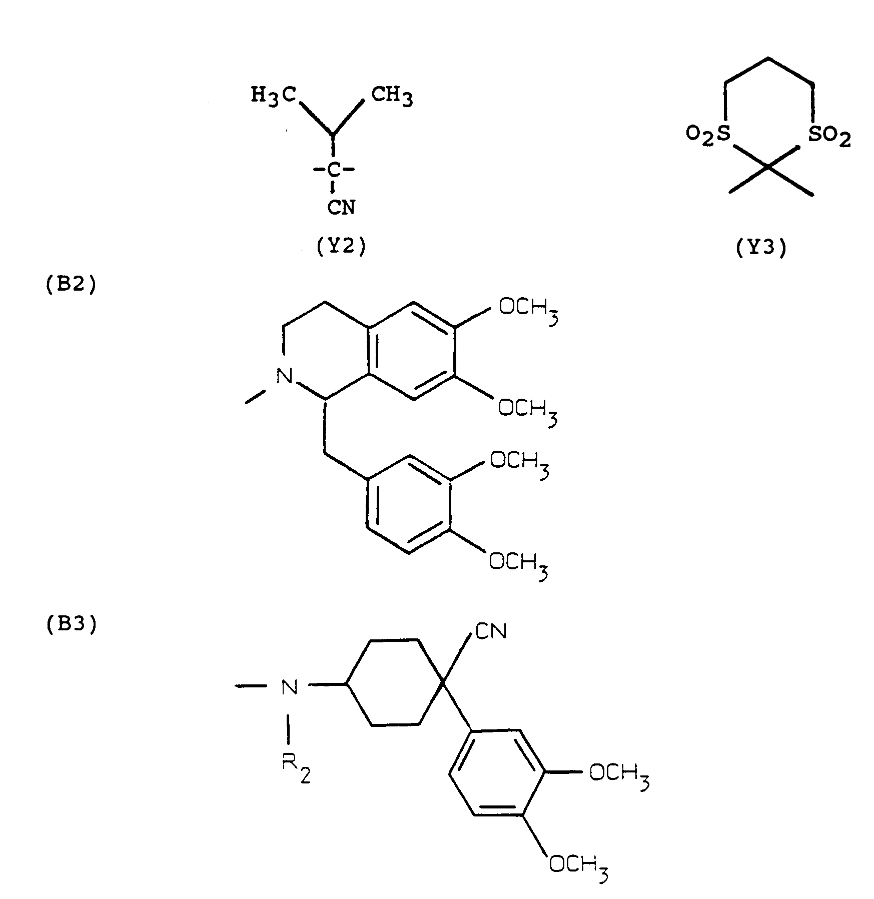 wherein R₂ is as above defined.

The invention also includes the enantiomers, diastereoisomers, N-oxides and pharmaceutically acceptable salts of these compounds.

The 3-methyl-4-oxo-2-phenyl-4H-1-benzopyran-8-yl group will be abbreviated hereinafter as Fl. The alphanumerics X1 through X12 and B1 through B3 will be used as abbreviations for the groups X and B to which they are respectively applied above.

X preferably represents one of the groups X2 or X3; p is preferably an integer of from 1 to 3; and B preferably represents the group B1, especially the 4-cyano-4-(3,4-dimethoxyphenyl)-5,N-dimethyl-hexylamino group.

The invention further provides a pharmaceutical composition comprising a compound according to the general formula I as above defined, or an enantiomer, diastereoisomer, N-oxide or pharmaceutically acceptable salt of such a compound, in admixture with a pharmaceutically acceptable diluent or carrier. Pharmaceutical compositions according to the invention may take the form of oral tablets, vaginal or uterine tablets or suppositories, pills, capsules, liquid solutions, suspensions, nasal sprays and the like. They are preferably presented in unit dosage forms suitable for simple administration of precise dosages. For oral administration, a daily dose of from 10 to 100 mg of the active compound will generally be suitable , either as a single daily dose or as up to three or four smaller dosages given regularly during the day. For i.v. administration, a daily dose of from 0.5 to 5 mg of the active compound will be suitable. The amount of active compound administered will, of course, depend upon its relative activity.

### PREPARATION OF THE COMPOUNDS OF THE INVENTION

The compounds according to the invention may generally be prepared by condensing compounds Fl-X-(CH₂)ₙ-Hal, wherein Hal represents a halogen atom, preferably a chlorine or bromine atom, with a compound H-B. The condensation is preferably, but not necessarily, carried out in a solvent such as dimethylformamide or methanol, usually in the presence of a base such as potassium carbonate. Such condensations are illustrated in Examples 1, 4 and 5 to 20 hereinbelow.

An alternative method for the preparation of the compounds according to the invention is the condensation of a compound Fl-X-H with a compound Hal-(CH₂)ₙ-B wherein Hal is as above defined. This condensation may be carried out under the conditions described in the last preceding paragraph, and is illustrated in Example 2 hereinbelow. In some cases, the compounds of the invention may be prepared by conversion of other compounds of the invention or by the N-oxidation reactions illustrated in Examples 3 and 5 hereinbelow. Yet other methods will be apparent to those skilled in the art.

The starting materials Fl-X-(CH₂)ₙ-Hal and Fl-X-H used in the above preparations may themselves be prepared from available compounds such as Fl-COOH, Fl-COONa, Fl-COCl and Fl-CHO by transformations known to those skilled in the art. Numerous such transformations are described in detail hereinbelow.

### DETAILED PREPARATION OF INTERMEDIATES

### 8-(3-bromopropoxycarbonyl)-3-methyl-4-oxo-2-phenyl-4H-1-benzopyran (Intermediate I)

104 g of 8-carboxy-3-methyl-4-oxo-2-phenyl-4H-1-benzopyran (prepared as described in P. Da Re et al., *J. Med. Pharm. Chem.,* 2, 263, 1960) was dissolved in 560 ml of hot methanol and 280 ml of an aqueous solution containing 31 g of sodium hydrogen carbonate was added. 850 ml of acetone was added, resulting in the precipitation of sodium 3-methyl-4-oxo-2-phenyl-4H-1-benzopyran-8-carboxylate which was collected by suction filtration. Yield 62 g; m.p. >280°C.

30 g of 1,3-dibromopropane was added dropwise at ambient temperature to a suspension of 30 g of sodium 3-methyl-4-oxo-2-phenyl-4H-1-benzopyran-8-carboxylate in 150 ml of dimethylformamide and 35 ml of water. The reaction mixture was stirred at ambient temperature for 5 days. 100 ml of water was added and stirring was continued for a further 15 minutes. The precipitate was filtered off by suction, washed with water and purified by flash chromatography on silica gel, eluting with chloroform:ethyl acetate 95:5. The collected fractions were evaporated to dryness in vacuo and the residue was recrystallized from ethanol to give 27.7 g of the title compound, m.p. 114-115°C.

### 8-hydroxymethyl-3-methyl-4-oxo-2-phenyl-4H-1-benzopyran (Intermediate II)

467 ml of a 1.48N solution of sodium borohydride in anhydrous dimethylformamide was added over a period of 30 minutes, under stirring at ambient temperature, to a solution of 100 g of 3-methyl-4-oxo-2-phenyl-4H-1-benzopyran-8-carbonyl chloride (prepared as described in P. Da Re et al., supra) in 1 litre of anhydrous dimethylformamide. The reaction mixture was stirred for 2½ hours at ambient temperature. 88 ml of 2N hydrochloric acid was added while maintaining the temperature at 0-5°C. 102 ml of 12.7N sodium hydroxide solution was then added. The mixture was poured into 6 litres of water, stirred for 3 hours, and filtered under suction. The filter cake was washed with 4N sodium hydroxide solution and then with water. The resultant white solid was crystallized from methanol to give 50 g of the title compound, m.p. 145-147°C.

### 8-acetyl-3-methyl-4-oxo-2-phenyl-4H-1-benzopyran (Intermediate III)

1.17 g of magnesium turnings, 7.4 ml of anhydrous ethanol and 0.2 ml of anhydrous carbon tetrachloride were placed in a round bottomed flask under a stream of nitrogen. When the temperature began to rise, 7.5 ml of anhydrous chlorobenzene was added, followed by the slow dropping (25 minutes) of a solution of 5.28 ml of anhydrous diethylmalonate and 3.5 ml of anhydrous chlorobenzene in 16 ml of anhydrous ethanol. The reaction flask was heated to 73°C for two hours, cooled to 25°C and a solution of 8.8 g of 3-methyl-4-oxo-2-phenyl-4H-1-benzopyran-8-carbonyl chloride in 88 ml of anhydrous chlorobenzene was slowly added, without exceeding 35°C. The reaction mixture was further stirred for two hours at 35°C and then cooled to 0°C. 13 ml of water and 1.9 ml of sulphuric acid (d = 1.34) were added. The solution obtained was decanted from the insoluble inorganic matter and stripped in vacuo.

The crude acylmalonate obtained was refluxed for six hours with 10.4 ml of acetic acid, 7 ml of water and 1.3 ml of sulphuric acid (d = 1.84). After cooling, the solution was poured on to iced water and the precipitate was collected by suction filtration and washed with aqueous sodium carbonate. Crystallization from 90% ethanol gave 6.5 g of the title compound, m.p. 159-161°C.

### 8-bromoacetyl-3-methyl-4-oxo-2-phenyl-4H-1-benzopyran (Intermediate IV)

A solution of 11.2 g of bromine in 250 ml of chloroform was added, over a period of two hours at 20-25°C, to a solution of 19.5 g of the Intermediate III in 700 ml of chloroform. After stirring for 1 hour at 20-25°C, the solution was washed with 400 ml of 2N aqueous sodium hydroxide solution and then repeatedly with water, dried with anhydrous sodium sulphate and stripped in vacuo. The crude product was treated with diethyl ether, collected by suction filtration and crystallized from acetone, yielding 16 g of the title compound, m.p 134-135°C.

### 8-(2-hydroxyethylcarbamoyl)-3-methyl-4-oxo-2-phenyl-4H-1-benzopyran (Intermediate V)

The title compound was prepared in the same manner as Intermediate XX, but using 2-aminoethanol instead of 3-aminopropanol. M.p. 206-208°C.

### 8-[N-(2-hydroxyethyl)-N-methyl-carbamoyl]-3-methyl-4-oxo-2-phenyl-4H-1-benzopyran (Intermediate VI)

A solution of 1.6 ml of 2-methylamino-ethanol in 10 ml of water was added dropwise over a period of 5 minutes to a suspension of 6 g of 3-methyl-4-oxo-2-phenyl-4H-1-benzopyran-8-carbonyl chloride and 1.52 g of potassium carbonate in 60 ml of acetone. After stirring for 2½ hours at 20-25°C, the solvent was removed in vacuo and the residue was taken up in 150 ml of acetone. The mixture was refluxed for 15 minutes, and was then filtered. The solvent was evaporated from the filtrate and the residue was dissolved in 20 ml of dimethylformamide, treated with 14 ml of 1.4% sodium carbonate solution, stirred for 30 minutes at 20-25°C and diluted by addition of 150 ml of water. The mixture was extracted with chloroform and the organic layer was washed with 0.5N hydrochloric acid and then with water. The solution was dried over anhydrous sodium sulphate and the chloroform was evaporated off. The resulting oil was taken up in 200 ml of diethyl ether and stirred for 2 hours at 20-25°C. The solids were collected by filtration and crystallized from ethyl acetate to give 2.97 g of the title compound, m.p. 128-130°C.

### 8-(2-chloroethylcarbamoyl)-3-methyl-4-oxo-2-phenyl-4H-1-benzopyran (Intermediate VII)

The title compound was prepared in the same manner as Intermediate XXI, but using Intermediate V in place of Intermediate XX and carrying out the reaction at ambient temperature. M.p. 181-182°C (ethyl acetate).

### 8-(N-methyl-2-chloroethylcarbamoyl)-3-methyl-4-oxo-2-phenyl-4H-1-benzopyran (Intermediate VIII)

A solution of 1.1 ml of thionyl chloride in 2 ml of dichloromethane was added to a solution of 3.37 g of Intermediate VI in 20 ml of dichloromethane, and the mixture was stirred for 4 hours at ambient temperature. Removal of the solvent in vacuo gave an oil which was taken up in diethyl ether. 3 g of the title compound precipitated as a white solid which was filtered off for use without further purification. M.p. 126-128°C (diethyl ether).

### 8-(2-chloroethoxymethyl)-3-methyl-4-oxo-2-phenyl-4H-1-benzopyran (Intermediate IX)

16 ml of thionyl chloride in 18 ml of chloroform was added at 0°C to a stirred solution of 23 g of Intermediate XIII and 11 ml of triethylamine in 185 ml of chloroform. The reaction mixture was warmed to 70°C and stirred for 2 hours. After cooling to ambient temperature, it was poured into water. The organic layer was separated, washed with sodium chloride solution, dried on anhydrous sodium sulphate and evaporated to dryness in vacuo. Yield 24 g of the title compound. A sample crystallized from ethanol had a melting point of 102-103°C.

### 8-chloromethyl-3-methyl-4-oxo-2-phenyl-4H-1-benzopyran (Intermediate X)

53.4 g of Intermediate II and 38.8 ml of anhydrous triethylamine were dissolved in 440 ml of chloroform. Into this solution, maintained at -10 to -2°C, there was dropped a solution of 19.8 ml of thionyl chloride in 80 ml of anhydrous chloroform. The reaction mixture was stirred at room temperature for 4 hours, and then diluted with 400 ml of water. The aqueous phase was extracted with chloroform, and the extracts were added to the chloroform phase. The chloroform solution was washed with brine, dried on anhydrous sodium sulphate and evaporated to dryness in vacuo. Yield: 56 g of the title compound, which on recrystallization from ethanol was shown to have a melting point of 112-113°C.

### 8-methylaminomethyl-3-methyl-4-oxo-2-phenyl-4H-1-benzopyran (Intermediate XI)

A solution of 15.1 g of anhydrous zinc chloride and 14.5 g of sodium cyanoborohydride in 400 ml of anhydrous methanol was added dropwise at 0°C into a stirred mixture of 8-formyl-3-methyl-4-oxo-2-phenyl-4H-1-benzopyran (prepared as described in K. Uneyama et al., *Bull. Chem. Soc. Jap.,* 58, 2361, 1985), 60.7 g of methylamine hydrochloride and 125 ml of triethylamine in 600 ml of anhydrous methanol. After stirring for 5 hours at 20-25°C, the solvent was evaporated off in vacuo and the residue was taken up in 200 ml of water and collected by suction filtration. The crude product was dissolved in aqueous acetic acid, washed with ethyl acetate and reprecipitated by addition of cold 6N sodium hydroxide solution. 49 g of the title compound was obtained. m.p. 97-99°C, after crystallization from 75% ethanol.

### 8-(2-chloroethylthiomethyl)-3-methyl-4-oxo-2-phenyl-4H-1-benzopyran (Intermediate XII)

A solution of 37 g of Intermediate X and 10.5 g of thiourea in 370 ml of ethanol was refluxed for 1 hour. The reaction mixture was cooled to ambient temperature, and 42 g of 8-amidinothiomethyl-3-methyl-4-oxo-2-phenyl-4H-1-benzopyran spontaneously crystallized. A sample recrystallized from ethanol had a melting point of 233-235°C.

48 ml of 35% aqueous sodium hydroxide solution was added to a vigorously stirred suspension of 35 g of the compound thus prepared and 1.05 g of benzyl triethylammonium chloride in 440 ml of 1,2-dichloroethane. The mixture was stirred for 2½ hours and then poured into 300 ml of water. The aqueous layer was extracted with 1,2-dichloroethane and the extracts were added to the organic layer which was washed with sodium chloride solution, dried on anhydrous sodium sulphate, and evaporated to dryness in vacuo. The residue was crystallized from methanol, giving 22 g of the title compound, m.p. 82-83°C.

### 8-(2-hydroxyethoxymethyl)-3-methyl-4-oxo-2-phenyl-4H-1-benzopyran (Intermediate XIII)

A solution of 2.5 g of Intermediate X in 25 ml of xylene and 3 ml of dioxane was prepared. 0.15 g of sodium was dissolved in 3.10 ml of anhydrous ethylene glycol, and this solution was added dropwise at ambient temperature to the solution of Intermediate X. After refluxing for 5½ hours, the reaction mixture was cooled to ambient temperature and poured into 50 ml of water. It was extracted with dichloromethane, and the extract was washed with sodium chloride solution, dried on anhydrous sodium sulphate and evaporated to dryness in vacuo. The solid residue was crystallized from ethanol, giving 2.1 g of the title compound, m.p. 132-133°C.

### 8-aminomethyl-3-methyl-4-oxo-2-phenyl-4H-1-benzopyran (Intermediate XIV)

A mixture of 21 g of Intermediate XVII and 19 g of triphenylphosphine in 160 ml of tetrahydrofuran was stirred at ambient temperature for 8 hours. Thin layer chromatography showed the disappearance of Intermediate XVII. 3 ml of water was added, and stirring was continued for a further 24 hours. The solvents were removed on a rotary evaporator, and the residue was dissolved in water as its acetate. The aqueous solution was washed with ethyl acetate, made basic with 37% sodium hydroxide solution and filtered under suction. The filter cake was washed with water and dessicated to give 18 g of the title compound. The hydrochloride, recrystallized from ethanol, had a melting point of 256-258°C.

### 8-(2-chloroethylsulphonylmethyl)-3-methyl-4-oxo-2-phenyl-4H-1-benzopyran (Intermediate XV)

41.6 ml of aqueous 30% hydrogen peroxide was added dropwise at 40°C over a period of 20 minutes to a solution of 26.2 g of Intermediate XII in 300 ml of glacial acetic acid. The mixture was heated to 60°C, stirred at that temperature for 4½ hours, cooled to ambient temperature and poured into 60 ml of water. Suction filtration gave a filter cake which was washed with water and dried, yielding 29.4 g of the title compound. A sample was crystallized from ethanol. M.p. 158-161°C.

### 8-[N-methyl-N-(2-chloroethyl)-aminomethyl]-3-methyl-4-oxo-2-phenyl-4H-1-benzopyran (Intermediate XVI)

A mixture of 22 g of Intermediate XI, 66 ml of 1-bromo-2-chloroethane and 11 g of anhydrous potassium carbonate in 88 ml of dimethylformamide was stirred at 20-25°C for 12 hours. The reaction mixture was then poured into 600 ml of water and extracted with dichloromethane. The organic layer was washed with water, dried on anhydrous sodium sulphate and acidified with ethanolic hydrogen chloride. The solvent and the excess 1-bromo-2-chloroethane were distilled off in vacuo at 70-80°C. The residue was taken up in cold 1N aqueous sodium hydroxide solution and extracted with dichloromethane. The organic solution was washed with water, dried on anhydrous sodium sulphate and evaporated to dryness in vacuo at 25-30°C. The crude title product was purified by flash chromatography on silica gel, eluting with ethyl acetate:petroleum ether 7:3, to give 18 g of the title compound melting at 118-120°C after crystallization from ethanol.

### 8-azidomethyl-3-methyl-4-oxo-2-phenyl-4H-1-benzopyran (Intermediate XVII)

A mixture of 22.8 g of Intermediate X and 6.8 g of sodium azide in 110 ml of dimethylformamide was stirred for 3 hours at 100°C. After cooling to ambient temperature, 130 ml of water and 88 ml of ethanol were added to the reaction mixture. After 1 hour, the crystals were collected by suction filtration, washed with water, and dried. Yield: 22 g of the title compound. A sample recrystallized from ethanol had a melting point of 132-134°C.

### 8-(N-methyl-N-chloroacetyl-aminomethyl)-3-methyl-4-oxo-2-phenyl-4H-1-benzopyran (Intermediate XVIII)

A solution of 6 ml of chloroacetyl chloride in 60 ml of 1,2-dichloroethane was added dropwise at -5 to 0°C to a solution of 20 g of Intermediate XI and 10 ml of triethylamine in 200 ml of 1,2-dichloroethane. After stirring at 20-25°C for 2 hours, 150 ml of water was added to the reaction mixture and the phases were separated. The organic phase was washed with water and dried on anhydrous sodium sulphate. The solvent was removed in vacuo, and the residue was crystallized from ethanol to give 22.5 g of the title compound, m.p. 146-148°C.

### 8-chloroacetamidomethyl-3-methyl-4-oxo-2-phenyl-4H-1-benzopyran (Intermediate XIX)

A solution of 3.2 ml of chloroacetyl chloride in 32 ml of 1,2-dichloroethane was added dropwise, under stirring at -5°C, to a mixture of 10 g of Intermediate XIV and 5.5 ml of triethylamine in 80 ml of 1,2-dichloroethane. The reaction mixture was stirred at ambient temperature for 1 hour and then 150 ml of water was added. The phases were separated; the aqueous phase was extracted with 1,2-dichloroethane and the extracts were added to the organic phase which was then washed with a cold saturated solution of sodium bicarbonate, washed with water, dried on anhydrous sodium sulphate and evaporated to dryness in vacuo. The residue was crystallized from ethanol to give 10.7 g of the title compound, m.p. 152-155°C.

### 8-(3-hydroxypropylcarbamoyl)-3-methyl-4-oxo-2-phenyl-4H-1-benzopyran (Intermediate XX)

A solution of 7.6 ml of 3-aminopropanol in 50 ml of water was added dropwise over a period of 30 minutes to a suspension of 30 g of 3-methyl-4-oxo-2-phenyl-4H-1-benzopyran-8-carbonyl chloride and 15.2 g of potassium carbonate in 400 ml of acetone. The thick suspension was stirred for 3 hours at 20-25°C. The solvents were removed in vacuo and the residue was taken up in 300 ml of water. After stirring for 1 hour, the precipitate was collected by suction filtration and washed with water. The crude product was purified by crystallization from 95% ethanol and 23.8 g of the title compound were obtained, m.p. 191-193°C. An additional 4.7 g of the title compound was obtained by concentration in vacuo of the crystallization liquors.

### 8-(3-chloropropylcarbamoyl)-3-methyl-4-oxo-2-phenyl-4H-1-benzopyran (Intermediate XXI)

A solution of 1.1 ml of thionyl chloride in 2 ml of chloroform was added to a boiling solution of 3.37 g of Intermediate XX in 20 ml of chloroform. After stirring for 90 minutes under reflux, the solvent was removed in vacuo and the residue was crystallized from acetonitrile to give 3 g of pure title compound, m.p. 193-194°C.

### EXAMPLES

### Example 1

### 8-{2-[4-cyano-4-(3,4-dimethoxyphenyl)-5,N-dimethyl-hexylamino]-1-oxoethyl}-3-methyl-4-oxo-2-phenyl-4H-1-benzopyran hydrochloride hydrate

A solution of 7.84 g of 4-cyano-4-(3,4-dimethoxyphenyl)-5,N-dimethyl-hexylamine hydrochloride (prepared as described in H. Laguerre et al., *Eur. J. Med. Chem.,* 25, 351,1990) in 20 ml of anhydrous dimethylformamide was poured into a stirred suspension of 7.12 g of Intermediate IV and 6.08 g of anhydrous potassium carbonate in 100 ml of dimethylformamide at 0 to 5°C. Stirring was continued at ambient temperature for 2 hours, and the reaction mixture was then poured into iced water. The resultant precipitate was collected by suction filtration and dissolved in methyl acetate. Excess 6.7 N ethanolic hydrogen chloride was added to the solution, followed by diethyl ether. The precipitate was collected by filtration and recrystallized from ethyl acetate to give 2.7 g of the title compound, m.p. 120-125°C.

### Example 2

### 8-{3-[2-(3,4-dimethoxyphenyl)-N-methyl-ethylamino]-propoxycarbonyl}-3-methyl-4-oxo-2-phenyl-4H-1-benzopyran hydrochloride

A mixture of 8.4 g of 8-carboxy-3-methyl-4-oxo-2-phenyl-4H-1-benzopyran and 8.28 g of anhydrous potassium carbonate in 120 ml of dimethylformamide was stirred at 80-85°C for 30 minutes. After cooling to ambient temperature, 9.78 g of N-(3-chloropropyl)-2-(3,4-dimethoxyphenyl)-N-methyl-ethylamine hydrochloride (prepared as described in GB 1290625) was added. The mixture was stirred for 3½ hours at 80-85°C and then poured into 480 ml of water. The solution was extracted with diethyl ether and the organic layer was separated off, washed with water, and dried over anhydrous sodium sulphate. The solvent was evaporated off in vacuo, and the oily residue was dissolved in ethanol and acidified by addition of 5N isopropanolic hydrogen chloride. The title compound was collected by filtration and crystallized from methanol. Yield 12.58 g, m.p. 225-226°C.

### Example 3

### 8-{3-[2-(3,4-dimethoxyphenyl)-N-methyl-N-oxo-ethylamino]-propoxycarbonyl}-3-methyl-4-oxo-2-phenyl-4H-1-benzopyran hydrochloride

A solution of 2.1 g of m-chloroperbenzoic acid in 200 ml of dichloromethane was added dropwise over a period of 2 hours at 0 to 5 °C to a solution of 5.67 g of the compound prepared in Example 2 in 132 ml of dichloromethane. The mixture was stirred for 2 hours at 0 to 5°C and stood overnight at the same temperature. It was then concentrated to 40-45 ml by evaporation in vacuo, and acidified with a solution of 3.5N hydrogen chloride in diethyl ether. The salt was precipitated by addition of diethyl ether and the pasty crude product was taken up in acetone and crushed down. The solid was collected by filtration and crystallized from ethanol to give 5.72 g of the title compound, m.p. 142-144°C.

### Example 4

### 8-{3-[4-cyano-4-(3,4-dimethoxyphenyl)-N,5-dimethyl-hexylamino]-propoxycarbonyl}-3-methyl-4-oxo-2-phenyl-4H-1-benzopyran hydrochloride

A mixture of 12 g of Intermediate I, 8.6 g of 4-cyano-4-(3,4-dimethoxyphenyl)-5,N-dimethyl-hexylamine and 6.2 g of anhydrous potassium carbonate in 30 ml of anhydrous dimethylformamide was stirred at ambient temperature for 2 days. After pouring into cold water, the mother liquor was decanted and the pasty precipitate was dissolved in diethyl ether. The organic solution was washed with water until neutrality and dried over anhydrous sodium sulphate. The solvent was evaporated off in vacuo and the oily residue was chromatographed on silica gel, eluting with chloroform:methanol 98:2. The fractions containing the pure base of the title compound were pooled, and the solvents were removed in vacuo. The residue was dissolved in isopropanol and acidified with 5N isopropanolic hydrogen chloride. The salt, collected by suction filtration, was recrystallized from isopropanol to give 9.72 g of the title compound, m.p. 146-150°C.

### Example 5

### 8-{3-[4-cyano-4-(3,4-dimethoxyphenyl)-N,5-dimethyl-N-oxo-hexylamino]-propoxycarbonyl}-3-methyl-4-oxo-2-phenyl-4H-1-benzopyran hydrochloride 0.25 hydrate

This compound was prepared according to the method of Example 3, but starting from the compound prepared in Example 4 rather than that prepared in Example 2. The salt was collected by filtration and crystallized from methanol:diethyl ether 2:5. M.p. 148-151°C.

### Example 6

### 8-{3-[N-ethyl-3-(4-methoxyphenyl)-2-propylamino]-propoxycarbonyl}-3-methyl-4-oxo-2-phenyl-4H-1-benzopyran hydrochloride

This compound was prepared by the method of Example 4, but using N-ethyl-3-(4-methoxyphenyl)-2-propylamine (prepared as described in R. Fusco et al., *Il Farmaco,* 3, 125, 1948) in place of 4-cyano-4-(3,4-dimethoxyphenyl)-5,N-dimethyl-hexylamine. The reaction was carried out for 24 hours at ambient temperature and then 12 hours at 85-90°C. The title compound containing 2.24% of water was obtained, m.p. 152-155°C (isopropanol).

### Example 7

### 8-{3-[1-(3,4-dimethoxybenzyl)-6,7-dimethoxy-2H-1,2,3,4-tetrahydro-2-isoquinolinyl]-propoxycarbonyl}-3-methyl-4-oxo-2-phenyl-4H-1-benzopyran hydrochloride

This compound was prepared by the method of Example 4, but using 1-(3,4-dimethoxybenzyl)-6,7-dimethoxy-2H-1,2,3,4-tetrahydro-isoquinoline (prepared as described in J. B. Stenlake et al., *Eur. J. Med. Chem.,* 9, 133, 1974) in place of 4-cyano-4-(3,4-dimethoxyphenyl)-5,N-dimethyl-hexylamine. The reaction was carried out for 2 hours at ambient temperature and then 6 hours at 60°C. The crude base was purified by chromatography on silica gel using ethyl acetate:chloroform 4:1 as eluant. The salt was made in methanol:acetone 4:1 by addition of 3N methanolic hydrogen chloride. M.p. 220-222°C (80% ethanol). The title compound contained 1.83% of water.

### Example 8

### 8-〈3-{3-[2-(3,4-dimethoxyphenyl)-1,1,3,3-tetraoxo-1,3-dithian-2-yl]-N-methyl-propylamino}-propoxycarbonyl〉-3-methyl-4-oxo-2-phenyl-4H-1-benzopyran hydrochloride

This compound was prepared by the method of Example 4, but using 3-[2-(3,4-dimethoxyphenyl)-1,1,3,3-tetraoxo-1,3-dithian-2-yl]-N-methyl-propylamine (prepared as described in H. Ramuz, *Arzneim.-Forsch.,* 28, 2051, 1978) in place of 4-cyano-4-(3,4-dimethoxyphenyl)-5,N-dimethyl-hexylamine. The reaction was carried out for 12 hours at 60°C. The crude base was purified by flash chromatography on silica gel, using ethyl acetate:methanol graduated 7:3 to 1:1 as eluant. The hydrochloride salt was isolated in amorphous form and contained 1.1% of ethyl acetate. M.p. 140-145°C with decomposition.

### Example 9

### (Z)-8-{3-[4-cyano-4-(3,4-dimethoxyphenyl)-N-methyl-cyclohexylamino]-propoxycarbonyl}-3-methyl-4-oxo-2-phenyl-4H-1-benzopyran methanesulphonate

This compound was prepared by the method of Example 4, but using (Z)-4-cyano-4-(3,4-dimethoxyphenyl)-N-methyl-cyclohexylamine (prepared as described in S. Dei et al., *J. Med. Chem.,* 34, 2219, 1991) in place of 4-cyano-4-(3,4-dimethoxyphenyl)-5,N-dimethyl-hexylamine. The reaction was carried out at ambient temperature for 5 days. The crude base was purified by flash chromatography on silica gel, eluting with ethyl acetate:methanol 9:1. The fractions containing the base of the title compound were pooled, the solvents were removed in vacuo and the residue was dissolved in dichloromethane. The solution was acidified with methanesulphonic acid. The salt was precipitated by addition of diethyl ether and collected by filtration. M.p. 204-206°C (ethanol).

### Example 10

### (E)-8-{3-[4-cyano-4-(3,4-dimethoxyphenyl)-N-methyl-cyclohexylamino]-propoxycarbonyl}-3-methyl-4-oxo-2-phenyl-4H-1-benzopyran methanesulphonate

This compound was prepared by the method of Example 9, but using (E)-4-cyano-4-(3,4-dimethoxyphenyl)-N-methyl-cyclohexylamine in place of the Z-isomer. The reaction was carried out for 7 days. M.p. 194-196°C (dichloromethane:ethyl acetate).

### Example 11

### 8-{3-(4-cyano-4-(3,4-dimethoxyphenyl)-N,5-dimethyl-hexylamino]-propylcarbamoyl}-3-methyl-4-oxo-2-phenyl-4H-1-benzopyran hydrochloride hemihydrate

A mixture of 5.8 g of 4-cyano-4-(3,4-dimethoxyphenyl)-N,5-dimethyl-hexylamine and 3.56 g of Intermediate XXI was heated at 180°C for 2 hours. After cooling, the dark mass was purified by flash chromatography on silica gel, eluting with dichloromethane:methanol 100:3. The fractions containing the base of the title compound were pooled and evaporated to dryness in vacuo. The residue was dissolved in boiling ethanol, filtered, and acidified with 5N ethanolic hydrogen chloride. The solvent was evaporated off in vacuo, the residue was taken up in diethyl ether and the suspension was stirred for 5 hours. The amorphous salt was collected by filtration and dried at 50°C, 0.2 mm Hg for 30 hours to give 5.3 g of the title compound, m.p. 95-125°C.

| Elemental Analysis for C₃₇H₄₃N₃O₅.HCl.½H₂O | | | | | | | | | | |
|---|---|---|---|---|---|---|---|---|---|---|
| calculated | C | 67.82; | H | 6.92; | N | 6.41; | Cl | 5.41; | H₂O | 1.37 |
| found | | 67.44 | | 6.98 | | 6.36 | | 5.41 | | 1.33 |

### Example 12

### 8-{3-[2-(3,4-methoxyphenyl)-N-methyl-ethylamino]-propylcarbamoyl}-3-methyl-4-oxo-2-phenyl-4H-1-benzopyran hydrochloride 0.3-H₂O

This compound was prepared according to the method described in Example 11, but using 2-(3,4-dimethoxyphenyl)-N-methyl-ethylamine (prepared as described in W. L. Nelson et al., *J. Org. Chem.,* 52, 1390, 1987) instead of 4-cyano-4-(3,4-dimethoxyphenyl)-5,N-dimethyl-hexylamine. The reaction was carried out for 5 hours and the crude base obtained from the chromatographic purification was crystallized from ethanol. The hydrochloride was prepared from the crystallized base. M.p. 195-197°C.

### Example 13

### 8-{2-[4-cyano-4-(3,4-dimethoxyphenyl)-N,5-dimethyl-hexylamino]-ethylcarbamoyl}-3-methyl-4-oxo-2-phenyl-4H-1-benzopyran hydrochloride hemihydrate

A mixture of 3.41 g of Intermediate VII, 2.9 g of 4-cyano-4-(dimethoxyphenyl)-5,N-dimethyl-hexylamine, 2.75 g of anhydrous potassium carbonate and 1.66 g of potassium iodide in 30 ml of anhydrous dimethylformamide was stirred at 55-60°C for 30 hours and then at 70-80°C for 12 hours. The solvent was evaporated off in vacuo, and the residue was taken up in water and extracted with chloroform. The organic layer was separated form the aqueous layer, washed with water, and dried over anhydrous sodium sulphate. The solvent was evaporated off in vacuo and the residue was purified by flash chromatography on silica gel, eluting with dichloromethane:methanol 100:2. The fractions containing the base of the title compound were pooled and evaporated to dryness in vacuo. The residue was dissolved in 95% ethanol. The solution was filtered and acidified with 5N ethanolic hydrogen chloride. The solvent was evaporated off and the residue was dried at 78°C, 0.5 mm Hg for 8 hours to give 2.3 g of the title compound, m.p. 101-103°C.

### Example 14

### 8-{2-[4-cyano-4-(3,4-dimethoxyphenyl)-N,5-dimethyl-hexylamino]-N-methyl-ethylcarbamoyl}-3-methyl-4-oxo-2-phenyl-4H-1-benzopyran hydrochloride hydrate

This compound was prepared according to the method described in Example 13, using Intermediate VIII instead of Intermediate VII. The reaction was carried out at 100°C for 6 hours and the hydrochloride salt was prepared in anhydrous ethanol. The title compound was dried at ambient temperature at 0.5 mm Hg for 16 hours. M.p. 102-106°C.

### Example 15

### 8-{2-[4-cyano-4-(3,4-dimethoxyphenyl)-N,5-dimethyl-hexylamino]-N-methyl-ethylaminomethyl}-3-methyl-4-oxo-2-phenyl-4H-1-benzopyran hydrochloride hydrate

A mixture of 4.5 g of Intermediate XVI, 7.68 g of 4-cyano-4-(3,4-dimethoxyphenyl)-5,N-dimethyl-hexylamine and 1.23 g of potassium iodide in 100 ml of anhydrous dimethylformamide was stirred at 80°C for 5½ hours. The mixture was cooled to ambient temperature, poured into water and extracted with ethyl acetate. The combined extracts were washed brine, dried on anhydrous sodium sulphate and evaporated to dryness in vacuo. The residue was purified by flash chromatography on silica gel, eluting with ethyl acetate:methanol 9:1. The collected fractions containing the base of the title compound were evaporated to dryness in vacuo. The residue was dissolved in ethanol and 1 molar equivalent of 1N ethanolic hydrogen chloride was added. The ethanol was evaporated off in vacuo and the residue was stirred in isopropyl ether and then filtered off. Yield 3.5 g of the title compound, melting at 105°C.

### Example 16

### 8-{2-[4-cyano-4-(3,4-dimethoxyphenyl)-N,5-dimethyl-hexylamino]-1-oxo-ethylaminomethyl}-3-methyl-4-oxo-2-phenyl-4H-1-benzopyran hydrochloride dihydrate

A mixture of 3.5 g of Intermediate XIX, 3.64 g of 4-cyano-4-(3,4-dimethoxyphenyl)-5,N-dimethyl-hexylamine hydrochloride and 3.57 g of anhydrous potassium carbonate in 14 ml of anhydrous dimethylformamide was stirred at ambient temperature for 24 hours. The reaction mixture was poured into water and extracted with dichloromethane. The organic layer was washed with brine, dried on anhydrous sodium sulphate and evaporated to dryness in vacuo. The residue was crystallized from acetone to give the title compound as a base. This was dissolved in ethyl acetate and treated with excess 5N ethanolic hydrogen chloride. The solids were collected by filtration and recrystallized from ethanol to give 2.45 g of the title compound, m.p. 139-142°C.

### Example 17

### 8-{2-[4-cyano-4-(3,4-dimethoxyphenyl)-N,5-dimethyl-hexylamino]-N-methyl-1-oxo-ethylaminomethyl}-3-methyl-4-oxo-2-phenyl-4H-1-benzopyran hydrochloride hydrate

A mixture of 4 g of Intermediate XVIII, 3.4 g of 4-cyano-4-(3,4-dimethoxyphenyl)-5,N-dimethyl-hexylamine and 1.6 g of anhydrous potassium carbonate was stirred at ambient temperature for 3 hours. The reaction mixture was then diluted with water and extracted with dichloromethane. The organic layer, after washing with brine and drying over anhydrous sodium sulphate, was evaporated to dryness in vacuo. The crude oily residue obtained was purified by flash chromatography on silica gel, eluting with ethyl acetate:petroleum ether 8:2. The collected fractions, containing the base of the title compound, were evaporated to dryness in vacuo and the residue was dissolved in ethyl acetate. The solution was acidified with a 3N solution of hydrogen chloride in diethyl ether giving, after recrystallization from acetone, 3.25 g of the title compound, melting at 94-125°C with decomposition.

### Example 18

### 8-{2-[4-cyano-4-(3,4-dimethoxyphenyl)-5,N-dimethyl-hexylamino]-ethoxymethyl}-3-methyl-4-oxo-2-phenyl-4H-1-benzopyran hydrochloride hydrate

A mixture of 3.6 g of Intermediate IX, 3.2 g of 4-cyano-4-(3,4-dimethoxyphenyl)-5,N-dimethyl-hexylamine, 1.73 g of potassium iodide and 1.5 g of anhydrous potassium carbonate in 50 ml of anhydrous dimethylformamide was stirred at 95°C for 2 hours. The reaction mixture was cooled to ambient temperature, poured into water and extracted with ethyl acetate. The combined extracts were washed with a saturated solution of sodium thiosulphate and with brine and were dried on anhydrous sodium sulphate. The solution was evaporated to dryness in vacuo, and the oily residue was purified by flash chromatography on silica gel, eluting with ethyl acetate:methanol 97:3. The collected fractions were evaporated to dryness in vacuo and dissolved in diethyl ether. An excess of a 3N solution of hydrogen chloride in diethyl ether was added. The solvent was removed, and the residue was crystallized from ethyl acetate to give, 3.3 g of the title compound, m.p. 117-120°C.

### Example 19

### 8-{2-[4-cyano-4-(3,4-dimethoxyphenyl)-5,N-dimethyl-hexylamino]-ethylthiomethyl}-3-methyl-4-oxo-2-phenyl-4H-1-benzopyran hydrochloride

A mixture of 3.8 g of Intermediate XII, 3.5 g of 4-cyano-4-(3,4-dimethoxyphenyl)-5,N-dimethyl-hexylamine, 1.63 g of potassium carbonate and 1.92 g of potassium iodide in 38 ml of anhydrous dimethylformamide was stirred at 90°C for 2 hours. After cooling to ambient temperature, the reaction mixture was poured into water and extracted with ethyl acetate. The organic layer was washed with a saturated solution of sodium thiosulphate and then with brine, and was then dried over anhydrous sodium sulphate. The solvent was evaporated off in vacuo to give an oily residue which was purified by flash chromatography on silica gel, eluting with ethyl acetate:petroleum ether 8:2. The fractions containing the base of the title compound were collected and evaporated to dryness in vacuo. The residue was dissolved in diethyl ether and treated with a 3N solution of hydrogen chloride in diethyl ether to give, after filtration, 4.85 g of the title compound, m.p. 110-115°C.

### Example 20

### 8-{2-[4-cyano-4-(3,4-dimethoxyphenyl)-5,N-dimethyl-hexylamino]-ethylsulphonylmethyl}-3-methyl-4-oxo-2-phenyl-4H-1-benzopyran hydrochloride hemihydrate

A mixture of 3.9 g of Intermediate XV, 3.24 g of 4-cyano-4-(3,4-dimethoxyphenyl)-5,N-dimethyl-hexylamine and 0.73 g of anhydrous potassium carbonate in 40 ml of anhydrous dimethylformamide was stirred at ambient temperature for 3 hours. The reaction mixture was poured into water and filtered. The filter cake was washed with water and dried. The crude solid obtained was purified by flash chromatography on silica gel, eluting with petroleum ether:ethyl acetate 4:3 to give the title compound as its base. A solution of the base in acetone was treated with a 3N solution of hydrogen chloride in diethyl ether. The solvents were evaporated off and the residue was stirred in diethyl ether to give 3.8 g of the title compound, m.p. 206°C.

### PHARMACOLOGICAL TESTS

### Methodology

Female Albino Swiss mice [Crl: CD-1 (ICR) BR] with a body weight of 20-30 g and male Sprague Dawley rats [Crl: CD° BR] with a body weight of 200-300 g from the Charles River Farm, Italy, were used for the trials. The animals were housed with free access to food and water and kept on a forced light-darkness cycle at 22-24°C until the day of the trials.

### Acute toxicity:

The acute toxicity was evaluated in female Albino Swiss mice after intraperitoneal or oral administration. Four logarithmic scaled doses of the compounds were dissolved or suspended in 0.5% aqueous Methocel and administered at a volume of 10 ml/kg to groups of 4 mice/dose. Mortality was recorded 7 days after the administration.

### Data analysis:

The LD₅₀ values and their relevant confidence limits were calculated according to the method of Weil (*Biometrics,* 8, 249, 1952).

### Inhibition of K⁺-induced rat bladder contractions:

Male rats were killed by a blow on the head and the lower abdomen was opened along the midline; the urinary bladder was removed by a transverse cut above the trigone and cut in two fragments (20-30 mm long, 1-2 mm wide). Each fragment was suspended in an isolated organ bath containing 10 ml of a normal Tyrode solution (composition in mM : NaCl 137, KCl 2.68, MgCl₂ 1.11, CaCl₂ 1.8, NaH₂PO₄ 0.41, NaHCO₃ 11.9, glucose 5.55) and connected, under a steady 1 g load, to an isometric transducer (DY-1, Basile). The solution was gassed with a mixture of 95% O₂ and 5% CO₂ and warmed at 37°C. After a 60minutes equilibration the tension responses to a single KCl dose (bath concentration = 80 mM) inducing phasic and tonic contractions were measured under isometric conditions at 30 minute intervals. After recording one or more reproducible responses, a solution of the test drug was added to the bath (final concentration from 10⁻⁴ to 10⁻⁹ M) and 30 minutes later a new contraction was induced.

### Data analysis:

The experimental groups were made up of at least 2 preparations from different animals for each drug concentration tested. The IC₅₀ values of the inhibition of agonist-induced contractions were evaluated by linear regression analysis.

### Stability:

The stability at 37°C and physiological pH (phosphate buffer, pH 7.4) of the ester derivatives, Fl-X2-(CH₂)ₙ-B, was evaluated by chromatographic determination of the 8-carboxy-3-methyl-4-oxo-2-phenyl-4H-1-benzopyran resulting from any hydrolysis of the ester group. The stability of the other compounds of the invention was not investigated as they are known to be less susceptible to degradation than the esters.

### Results

The compounds prepared in the Examples were tested according to the above methods, and the results are shown in the following Table, together with comparative results obtained with Flavoxate. The data in the Table confirms that the compounds of the invention, with the exception of that of Example 1, are much more potent than Flavoxate in inhibiting the K⁺-induced rat bladder contractions, supporting a better spasmolytic effect for these compounds. In addition, most of the compounds are endowed with lower acute toxicity than Flavoxate; those which are not, e.g. the compounds of Examples 11 and 14, can nevertheless be considered safer than Flavoxate, as their therapeutic index (LD₅₀/IC₅₀) is higher. Finally, the tested esters proved to be much more stable than Flavoxate at physiological pH. Although, based on the results in the Table, the N-oxide compounds (Example 3 and 5) do not appear to be very active, they might be useful as prodrugs of more active compounds (see for instance: J.P. Gorrod et al., *Xenobiotica,* 3, 341, 1973; M. Strolin *Benedetti, Actual. Chim. Ther.,* 18th Series, page 243, 1991).

**TABLE**

| Compound Example No. | Inhibition of K⁺ induced rat bladder contractions IC₅₀ (»M) | | Mice LD₅₀ (mg/kg) | | Stability at pH 7.4 % Unchanged after 3 hours |
|---|---|---|---|---|---|
| | Phasic | Tonic | i.p. | p.o. | |
| 1 | 10.0 | >10.0 | 475 | >3000 | |
| 2 | 1.0 | 0.5 | 773 | 2255 | |
| 3 | >10.0 | >10.0 | >1000 | >3000 | |
| 4 | 0.5 | 1.3 | >1000 | >3000 | 100 |
| 5 | 10.0 | 2.2 | >1000 | >3000 | |
| 6 | 1.7 | 4.2 | >1000 | >3000 | 99 |
| 7 | 1.4 | 0.9 | >1000 | >3000 | 99 |
| 11 | 0.6 | 0.5 | 224 | 400 | |
| 12 | 2.2 | 1.4 | 129 | 716 | |
| 13 | 0.3 | 0.1 | 422 | | |
| 14 | 0.1 | 0.1 | 159 | 346 | |
| 16 | 0.5 | 0.9 | 440 | 1280 | |
| 17 | 0.2 | 0.4 | 334 | 1714 | |
| 18 | 0.3 | 0.4 | 334 | 974 | |
| 19 | 0.5 | 1.3 | >1000 | >3000 | |
| 20 | 0.5 | 0.5 | >1000 | >3000 | |
| Flavoxate | 13.0 | 13.0 | 385 | 808 | 10 |

## Claims

1. A compound having the general formula I 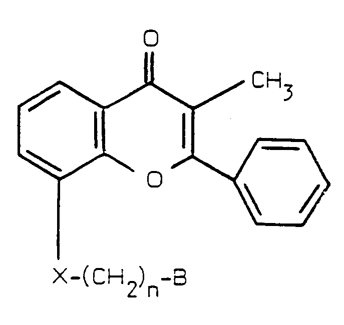 wherein
X represents one of the following groups, each of which is depicted with its left hand end being the end which attaches to the benzopyran ring and its right hand end being the end which attaches to the alkylene chain:
(X1) -CO-,
(X2) -COO-,
(X3) -CONH-,
(X4) -CON(CH₃)-,
(X5) -CH₂NH-,
(X6) -CH₂N(CH₃)-,
(X7) -CH₂NHCO-,
(X8) -CH₂N(CH₃)CO-,
(X9) -CH₂O-,
(X10) -CH₂S-,
(X11) -CH₂SO-, and
(X12) -CH₂SO₂-,
n is an integer of from 1 to 4; and
B represents one of the following groups: 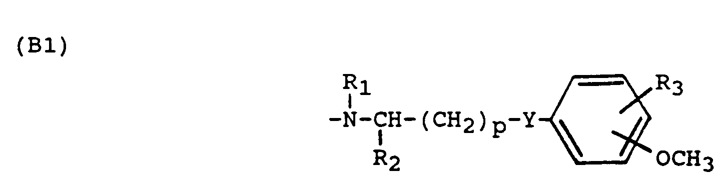 wherein R₁ represents an alkyl group having from 1 to 4 carbon atoms, R₂ represents a hydrogen atom or an alkyl group having from 1 to 4 carbon atoms, R₃ represents a hydrogen atom or a methoxy group, p is an integer of from 1 to 3, and Y represents (Y1) a valence bond or one of the following groups 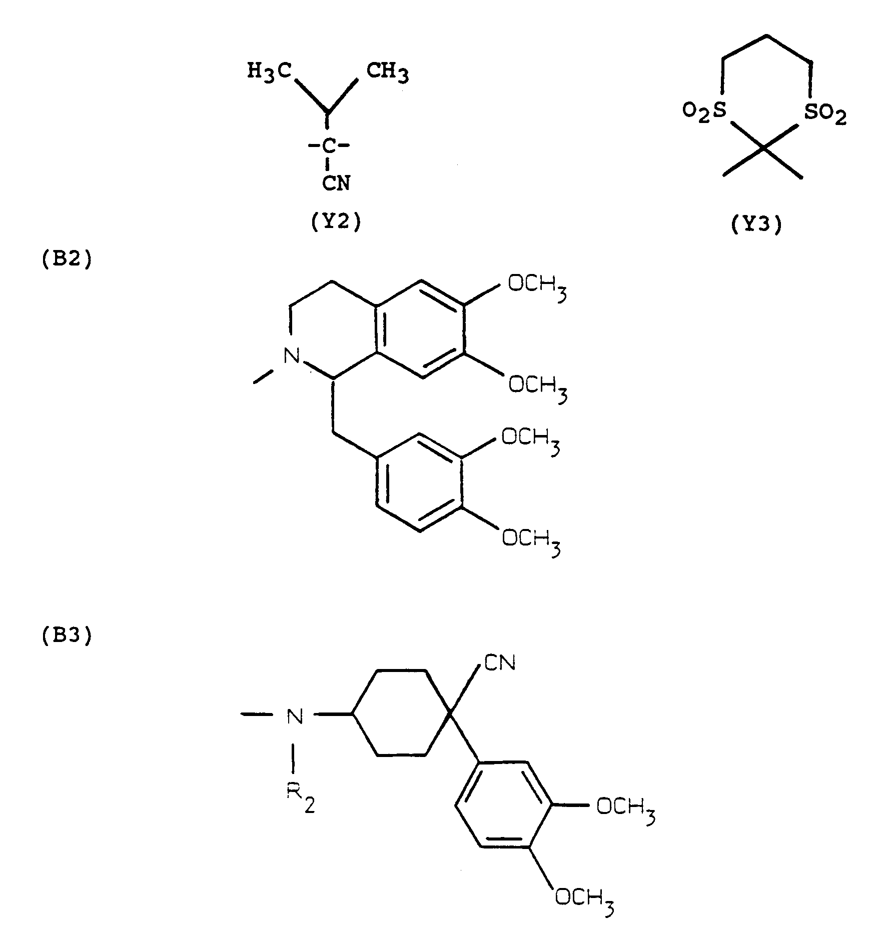 wherein R₂ is as defined in this claim,
or an enantiomer, diastereoisomer, N-oxide or pharmaceutically acceptable salt of such a compound.

2. A compound according to claim 1 wherein X represents one of the groups X2 or X3.

3. A compound according to claim 1 or claim 2 wherein n represents a integer of from 1 to 3.

4. A compound according to any preceding claim wherein B represents a group B1.

5. A compound according to claim 4 wherein B represents a 4-cyano-4-(3,4-dimethoxyphenyl)-N,5-dimethyl-hexylamino group.

6. A compound according to claim 1, being any one of the following:
8-{2-[4-cyano-4-(3,4-dimethoxyphenyl)-5,N-dimethyl-hexylamino]-1-oxoethyl}-3-methyl-4-oxo-2-phenyl-4H-1-benzopyran,
8-{3-[2-(3,4-dimethoxyphenyl)-N-methyl-ethylamino]-propoxycarbonyl}-3-methyl-4-oxo-2-phenyl-4H-1-benzopyran,
8-{3-[2-(3,4-dimethoxyphenyl)-N-methyl-N-oxo-ethylamino]-propoxycarbonyl}-3-methyl-4-oxo-2-phenyl-4H-1-benzopyran,
8-{3-[4-cyano-4-(3,4-dimethoxyphenyl)-N,5-dimethyl-hexylamino]-propoxycarbonyl}-3-methyl-4-oxo-2-phenyl-4H-1-benzopyran,
8-{3-[4-cyano-4-(3,4-dimethoxyphenyl)-N,5-dimethyl-N-oxo-hexylamino]-propoxycarbonyl}-3-methyl-4-oxo-2-phenyl-4H-1-benzopyran,
8-{3-[N-ethyl-3-(4-methoxyphenyl)-2-propylamino]-propoxycarbonyl}-3-methyl-4-oxo-2-phenyl-4H-1-benzopyran,
8-{3-[1-(3,4-dimethoxybenzyl)-6,7-dimethoxy-2H-1,2,3,4-tetrahydro-2-isoquinolinyl]-propoxycarbonyl)-3-methyl-4-oxo-2-phenyl-4H-1-benzopyran,
8-〈3-{3-[2-(3,4-dimethoxyphenyl)-1,1,3,3-tetraoxo-1,3-dithian-2-yl]-N-methyl-propylamino}-propoxycarbonyl〉-3-methyl-4-oxo-2-phenyl-4H-1-benzopyran,
(Z)-8-{3-[4-cyano-4-(3,4-dimethoxyphenyl)-N-methyl-cyclohexylamino]-propoxycarbonyl}-3-methyl-4-oxo-2-phenyl-4H-1-benzopyran,
(E)-8-{3-[4-cyano-4-(3,4-dimethoxyphenyl)-N-methyl-cyclohexylamino]-propoxycarbonyl}-3-methyl-4-oxo-2-phenyl-4H-1-benzopyran,
8-{3-[4-cyano-4-(3,4-dimethoxyphenyl)-N,5-dimethyl-hexylamino]-propylcarbamoyl}-3-methyl-4-oxo-2-phenyl-4H-1-benzopyran,
8-{3-[2-(3,4-methoxyphenyl)-N-methyl-ethylamino]-propylcarbamoyl}-3-methyl-4-oxo-2-phenyl-4H-1-benzopyran,
8-{2-[4-cyano-4-(3,4-dimethoxyphenyl)-N,5-dimethyl-hexylamino]-ethylcarbamoyl}-3-methyl-4-oxo-2-phenyl-4H-1-benzopyran,
8-{2-[4-cyano-4-(3,4-dimethoxyphenyl)-N,5-dimethyl-hexylamino]-N-methyl-ethylcarbamoyl}-3-methyl-4-oxo-2-phenyl-4H-1-benzopyran,
8-{2-[4-cyano-4-(3,4-dimethoxyphenyl)-N,5-dimethyl-hexylamino]-N-methyl-ethylaminomethyl}-3-methyl-4-oxo-2-phenyl-4H-1-benzopyran,
8-{2-[4-cyano-4-(3,4-dimethoxyphenyl)-N,5-dimethyl-hexylamino]-1-oxo-ethylaminomethyl}-3-methyl-4-oxo-2-phenyl-4H-1-benzopyran,
8-{2-[4-cyano-4-(3,4-dimethoxyphenyl)-N,5-dimethyl-hexylamino]-N-methyl-1-oxo-ethylaminomethyl}-3-methyl-4-oxo-2-phenyl-4H-1-benzopyran,
8-{2-[4-cyano-4-(3,4-dimethoxyphenyl)-5,N-dimethyl-hexylamino]-ethoxymethyl}-3-methyl-4-oxo-2-phenyl-4H-1-benzopyran,
8-{2-[4-cyano-4-(3,4-dimethoxyphenyl)-5,N-dimethyl-hexylamino]-ethylthiomethyl}-3-methyl-4-oxo-2-phenyl-4H-1-benzopyran, and
8-{2-[4-cyano-4-(3,4-dimethoxyphenyl)-5,N-dimethyl-hexylamino]-ethylsulphonylmethyl}-3-methyl-4-oxo-2-phenyl-4H-1-benzopyran.

7. A pharmaceutical composition comprising a compound according to any preceding claim, or an enantiomer, diastereoisomer, N-oxide or pharmaceutically acceptable salt of such a compound, in admixture with a pharmaceutically acceptable diluent or carrier.

## Patentansprüche

1. Verbindung der allgemeinen Formel I 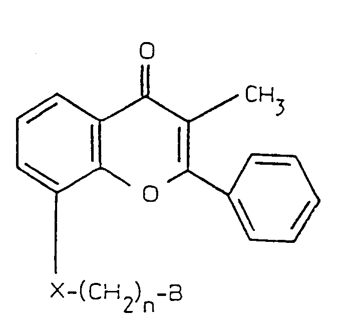 wobei
X eine der folgenden Gruppen darstellt, von denen jede mit ihrem linken Ende mit dem Benzopyranring verbunden und mit ihrem rechten Ende mit der Alkylenkette verbunden dargestellt ist:
(X1) -CO-,
(X2) -COO-,
(X3) -CONH-,
(X4) -CON(CH₃)-,
(X5) -CH₂NH-,
(X6) -CH₂N(CH₃)-,
(X7) -CH₂NHCO-,
(X8) -CH₂N(CH₃)CO-,
(X9) -CH₂O-,
(X10) -CH₂S-,
(X11) -CH₂SO-, und
(X12) -CH₂SO₂,
n eine ganze Zahl von 1 bis 4 ist; und
B eine der folgenden Gruppen darstellt: 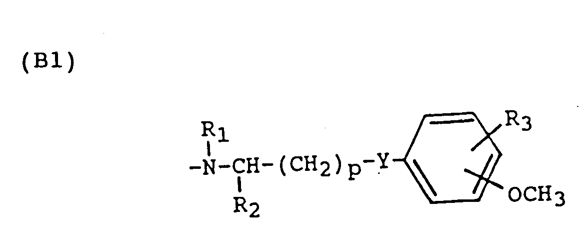 wobei R₁ eine Alkylgruppe mit 1 bis 4 Kohlenstoffatomen darstellt, R₂ ein Wasserstoffatom oder eine Alkylgruppe mit 1 bis 4 Kohlenstoffatomen darstellt, R₃ ein Wasserstoffatom oder eine Methoxygruppe darstellt, p eine ganze Zahl von 1 bis 3 ist und Y eine Valenzbindung (Y1) oder eine der folgenden Gruppen darstellt 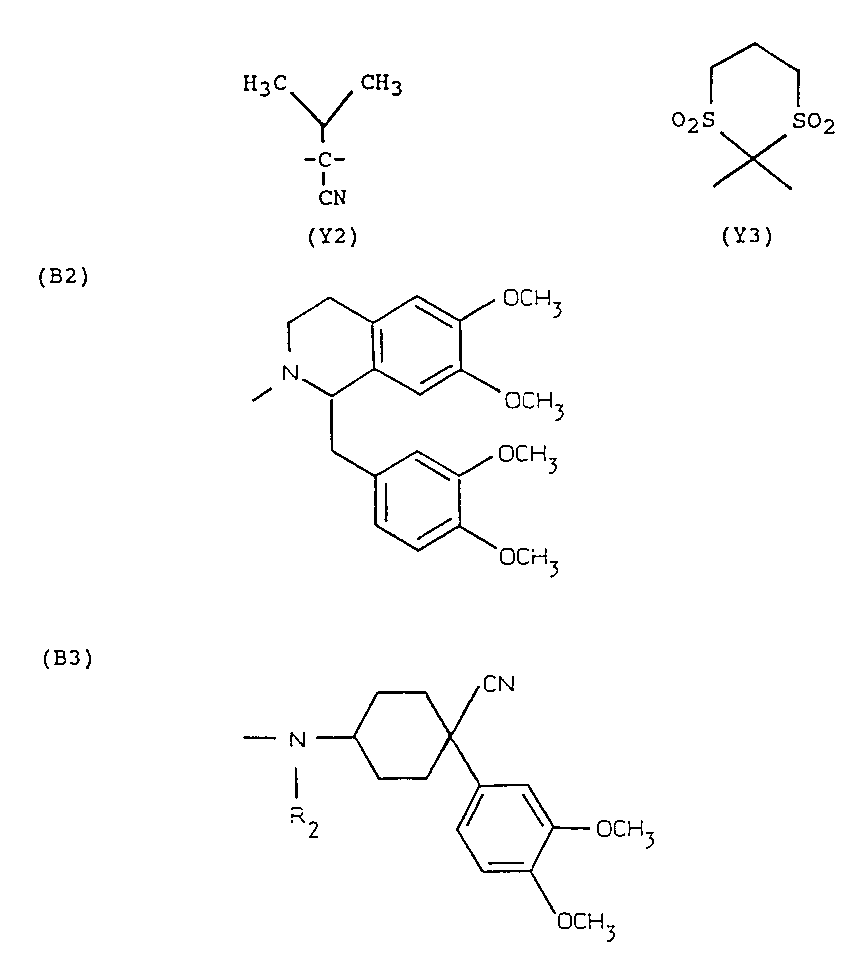 wobei R₂ ein Wasserstoffatom oder eine Alkylgruppe mit 1 bis 4 Kohlenstoffatomen darstellt,
oder ein Enantiomer, Diastereomer, N-Oxid oder pharmazeutisch akzeptables Salz einer solchen Verbindung.

2. Verbindung nach Anspruch 1, wobei X eine der Gruppen X2 oder X3 darstellt.

3. Verbindung nach Anspruch 1 oder 2, wobei n eine ganze Zahl von 1 bis 3 ist.

4. Verbindung nach einem der vorhergehenden Ansprüche, wobei B eine Gruppe B1 darstellt.

5. Verbindung nach Anspruch 4, wobei B eine 4-Cyano-4-(3,4-dimethoxyphenyl)-N,5-dimethyl-hexylamino-Gruppe darstellt.

6. Verbindung nach Anspruch 1, wobei diese eine der folgenden Verbindungen ist:
8-(2-[4-Cyano-4-(3,4-dimethoxyphenyl)-5,N-dimethyl-hexylamino]-1-oxoethyl)-3-methyl-4-oxo-2-phenyl-4H-1-benzopyran,
8-(3-[2-(3,4-Dimethoxyphenyl)-N-methyl-ethylamino]-propoxycarbonyl)-3-methyl-4-oxo-2-phenyl-4H-1-benzopyran,
8-(3-[2-(3,4-Dimethoxyphenyl)-N-methyl-N-oxo-ethylamino]-propoxycarbonyl)-3-methyl-4-oxo-2-phenyl-4H-1-benzopyran,
8-(3-[4-Cyano-4-(3,4-dimethoxyphenyl)-N,5-dimethyl-hexylamino]-propoxycarbonyl)-3-methyl-4-oxo-2-phenyl-4H-1-benzopyran,
8-(3-[4-Cyano-4-(3,4-dimethoxyphenyl)-N,5-dimethyl-N-oxo-hexylamino]-propoxycarbonyl)-3-methyl-4-oxo-2-phenyl-4H-1-benzopyran,
8-(3-[N-Ethyl-3-(4-methoxyphenyl)-2-propylamino]-propoxycarbonyl)-3-methyl-4-oxo-2-phenyl-4H-1-benzopyran,
8-(3-[1-(3,4-Dimethoxybenzyl)-6,7-dimethoxy-2H-1,2,-3,4-tetrahydro-2-isochinolinyl]-propoxycarbonyl)-3-methyl-4-oxo-2-phenyl-4H-1-benzopyran,
8-(3-(3-[2-(3,4-Dimethoxyphenyl)-1,1,3,3-tetraoxo-1,3-dithian-2-yl]-N-methyl-propylamino)-propoxycarbonyl)-3-methyl-4-oxo-2-phenyl-4H-1-benzopyran,
(Z)-8-(3-[4-Cyano-4-(3,4-dimethoxyphenyl)-N-methyl-cyclohexylamino]-propoxycarbonyl)-3-methyl-4-oxo-2-phenyl-4H-1-benzopyran,
(E)-8-(3-[4-Cyano-4-(3,4-dimethoxyphenyl)-N-methyl-cyclohexylamino]-propoxycarbonyl)-3-methyl-4-oxo-2-phenyl-4H-1-benzopyran,
8-(3-[4-Cyano-4-(3,4-dimethoxyphenyl)-N,5-dimethyl-hexylamino]-propylcarbamoyl)-3-methyl-4-oxo-2-phenyl-4H-1-benzopyran,
8-(3-[2-(3,4-Methoxyphenyl)-N-methyl-ethylamino]-propylcarbamoyl)-3-methyl-4-oxo-2-phenyl-4H-1-benzopyran,
8-(2-[4-Cyano-4-(3,4-dimethoxyphenyl)-N,5-dimethyl-hexylamino]-ethylcarbamoyl)-3-methyl-4-oxo-2-phenyl-4H-1-benzopyran,
8-(2-[4-Cyano-4-(3,4-dimethoxyphenyl)-N,5-dimethyl-hexylamino]-N-methyl-ethylcarbamoyl)-3-methyl-4-oxo-2-phenyl-4H-1-benzopyran,
8-(2-[4-Cyano-4-(3,4-dimethoxyphenyl)-N,5-dimethyl-hexylamino]-N-methyl-ethylaminomethyl)-3-methyl-4-oxo-2-phenyl-4H-1-benzopyran,
8-(2-[4-Cyano-4-(3,4-dimethoxyphenyl)-N,5-dimethyl-hexylamino]-1-oxo-ethylaminomethyl)-3-methyl-4-oxo-2-phenyl-4H-1-benzopyran,
8-(2-[4-Cyano-4-(3,4-dimethoxyphenyl)-N,5-dimethyl-hexylamino]-N-methyl-1-oxo-ethylaminomethyl)-3-methyl-4-oxo-2-phenyl-4H-1-benzopyran,
8-(2-[4-Cyano-4-(3,4-dimethoxyphenyl)-5,N-dimethyl-hexylamino]-ethoxymethyl)-3-methyl-4-oxo-2-phenyl-4H-1-benzopyran,
8-(2-[4-Cyano-4-(3,4-dimethoxyphenyl)-5,N-dimethyl-hexylamino]-ethylthiomethyl)-3-methyl-4-oxo-2-phenyl-4H-1-benzopyran und
8-(2-[4-Cyano-4-(3,4-dimethoxyphenyl)-5,N-dimethyl-hexylamino]-ethylsulphonylmethyl)-3-methyl-4-oxo-2-phenyl-4H-1-benzopyran.

7. Pharmazeutische Zusammensetzung mit einer Verbindung nach einem der vorhergehenden Ansprüche oder einem Enantiomer, Diastereomer, N-Oxid oder pharmazeutisch akzeptablem Salz einer solchen Verbindung in einer Mischung mit einem pharmazeutisch akzeptablen Verdünnungsmittel oder Träger.

## Revendications

1. Un composé présentant la formule générale I 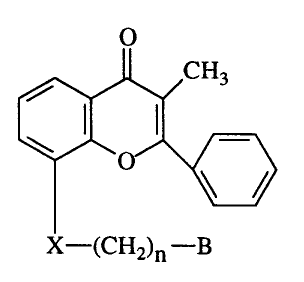 dans laquelle
X représente l'un des groupes suivants, chacun d'eux étant représenté avec son extrémité gauche constituant l'extrémité qui est fixée au noyau benzopyrane et son extrémité droite constituant l'extrémité qui est fixée à la chaîne alkylène :
(X1) -CO-,
(X2) -COO-,
(X3) -CONH-,
(X4) -CON(CH₃)-,
(X5) -CH₂NH-,
(X6) -CH₂N(CH₃)-,
(X7) -CH₂NHCO-,
(X8) -CH₂N(CH₃)CO-,
(X9) -CH₂O-,
(X10) -CH₂S-,
(X11) -CH₂SO-, et
(X12) -CH₂SO₂-,
n est un nombre entier de 1 à 4 ; et
B représente l'un des groupes suivants : 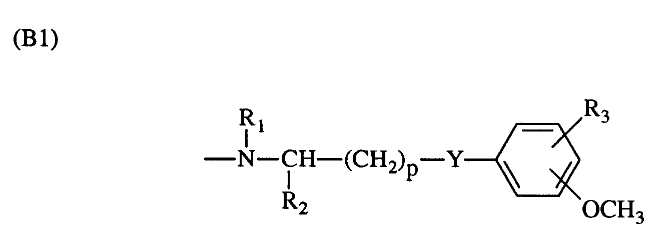 dans lequel R₁ représente un groupe alkyle comportant de 1 à 4 atomes de carbone, R₂ représente un atome d'hydrogène ou un groupe alkyle comportant de 1 à 4 atomes de carbone, R₃ représente un atome d'hydrogène ou un groupe méthoxy, p est un nombre entier de 1 à 3 et Y représente (Y1) une liaison de valence ou l'un des groupes suivants 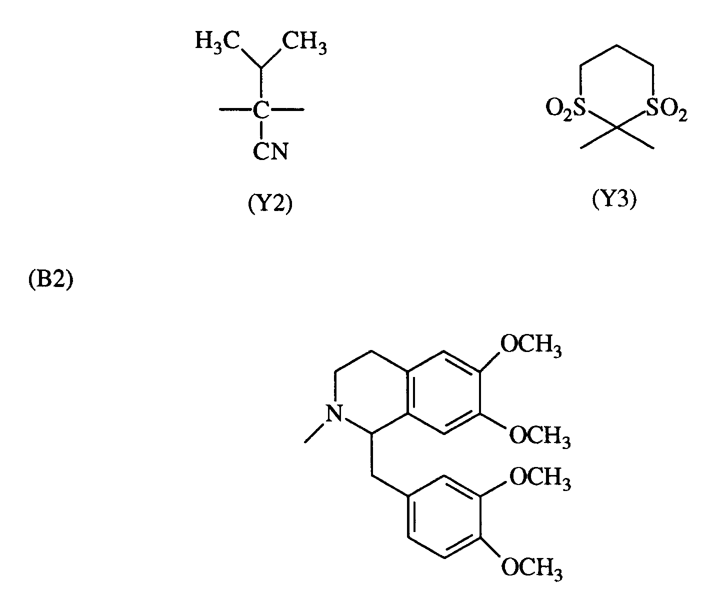 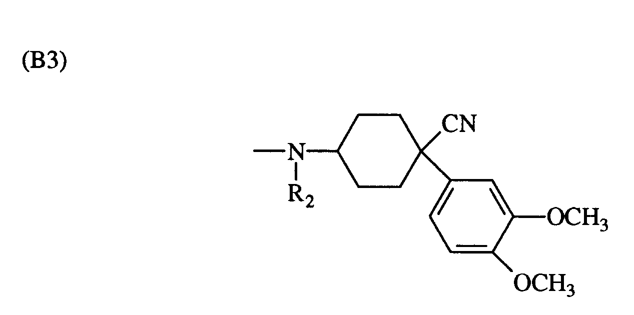 dans lequel R₂ est tel que défini dans cette revendication,
ou un énantiomère, diastéréoisomère, N-oxyde ou sel pharmaceutiquement acceptable d'un tel composé.

2. Un composé selon la revendication 1, dans lequel X représente l'un des groupes X2 ou X3.

3. Un composé selon la revendication 1 ou la revendication 2, dans lequel n représente un nombre entier de 1 à 3.

4. Un composé selon l'une quelconque des revendications précédentes, dans lequel B représente un groupe B1.

5. Un composé selon la revendication 4, dans lequel B représente un groupe 4-cyano-4-(3,4-diméthoxyphényl)-N,5-diméthyl-hexylamino.

6. Un composé selon la revendication 1, constituant l'un quelconque des suivants :
8-{2-[4-cyano-4-(3,4-diméthoxyphényl)-5,N-diméthyl-hexylamino]-1-oxoéthyl}-3-méthyl-4-oxo-2-phényl-4H-1-benzopyrane,
8-{3-[2-(3,4-diméthoxyphényl)-N-méthyl-éthylamino]-propoxycarbonyl}-3-méthyl-4-oxo-2-phényl-4H-1-benzopyrane,
8-{3-[2-3,4-diméthoxyphényl)-N-méthyl-N-oxo-éthylamino]-propoxycarbonyl}-3-méthyl-4-oxo-2-phényl-4H-1-benzopyrane,
8-{3-[4-cyano-4-(3,4-diméthoxyphényl)-N,5-diméthyl-hexylamino]-propoxycarbonyl}-3-méthyl-4-oxo-2-phényl-4H-1-benzopyrane,
8-{3-[4-cyano-4-(3,4-diméthoxyphényl)-n,5-diméthyl-N-oxo-hexylamino]-propoxycarbonyl}-3-méthyl-4-oxo-2-phényl-4H-benzopyrane,
8-{3-[N-éthyl-3-(4-méthoxyphényl)-2-propylamino]-propoxycarbonyl}-3-méthyl-4-oxo-2-phényl-4H-1-benzopyrane,
8-{3-[1-(3,4-diméthoxybenzyl)-6,7-diméthoxy-2H-1,2,3,4-tétrahydro-2-isoquinolinyl]-propoxycarbonyl}-3-méthyl-4-oxo-2-phényl-4H-1-benzopyrane,
8-〈3-{3-[2-(3,4-diméthoxyphényl)-1,1,3,3-tétraoxo-1,3-dithiane-2-yl]-N-méthyl-propylamino}-propoxycarbonyl〉-3-méthyl-4-oxo-2-phényl-4H-1-benzopyrane,
(Z)-8-{3-[4-cyano-4-(3,4-diméthoxyphényl)-N-méthyl-cyclohexylamino]-propoxycarbonyl}-3-méthyl-4-oxo-2-phényl-4H-1-benzopyrane,
(E)-8-{3-[4-cyano-4-(3,4-diméthoxyphényl)-N-méthyl-cyclohexylamino]-propoxycarbonyl}-3-méthyl-4-oxo-2-phényl-4H-1-benzopyrane,
8-{3-[4-cyano-4-(3,4-diméthoxyphényl)-N,5-diméthyl-hexylamino]-propylcarbamoyl}-3-méthyl-4-oxo-2-phényl-4H-1-benzopyrane,
8-{3-[2-(3,4-méthoxyphényl)-N-méthyl-éthylamino]-propylcarbamoyl}-3-méthyl-4-oxo-2-phényl-4H-1-benzopyrane,
8-{2-[4-cyano-4-(3,4-diméthoxyphényl)-N,5-diméthyl-hexylamino]-éthylcarbamoyl}-3-méthyl-4-oxo-2-phényl-4H-1-benzopyrane,
8-{2-[4-cyano-4-(3,4-diméthoxyphényl)-N,5-diméthyl-hexylamino]-N-méthyl-éthylcarbamoyl}-3-méthyl-4-oxo-2-phényl-4H-1-benzopyrane,
8-{2-[4-cyano-4-(3,4-diméthoxyphényl)-N,5-diméthyl-hexylamino]-N-méthyl-éthylaminométhyl}-3-méthyl-4-oxo-2-phényl-4H-1-benzopyrane,
8-{2-[4-cyano-4-(3,4-diméthoxyphényl)-N,5-diméthyl-hexylamino]-1-oxo-éthylaminométhyl}-3-méthyl-4-oxo-2-phényl-4H-1-benzopyrane,
8-{2-[4-cyano-4-(3,4-diméthoxyphényl)-N,5-diméthyl-hexylamino]-N-méthyl-1-oxo-éthylaminométhyl}-3-méthyl-4-oxo-2-phényl-4H-1-benzopyrane,
8-{2-[4-cyano-4-(3,4-diméthoxyphényl)-5,N-diméthyl-hexylamino]-éthoxyméthyl}-3-méthyl-4-oxo-2-phényl-4H-1-benzopyrane,
8-{2-[4-cyano-4-(3,4-diméthoxyphényl)-5,N-diméthyl-hexylamino]-éthylthiométhyl}-3-méthyl-4-oxo-2-phényl-4H-1-benzopyrane, et
8-{2-[4-cyano-4-(3,4-diméthoxyphényl)-5,N-diméthyl-hexylamino]-éthylsulfonylméthyl}-3-méthyl-4-oxo-2-phényl-4H-1-benzopyrane.

7. Une composition pharmaceutique comprenant un composé selon l'une quelconque des revendications précédentes, ou un énantiomère, diastéréoisomère, N-oxyde ou sel pharmaceutiquement acceptable d'un tel composé, en mélange avec un diluant ou un excipient pharmaceutiquement acceptable.
